**Europäisches Patentamt**

**European Patent Office**

**Office européen des brevets**

(19)

(11) Publication number : **0 326 278 B1**

(12)                     **EUROPEAN PATENT SPECIFICATION**

(45) Date of publication of patent specification :
**08.04.92 Bulletin 92/15**

(51) Int. Cl.[5] : **A61K 9/70, A61K 9/00**

(21) Application number : **89300448.1**

(22) Date of filing : **19.01.89**

(54) Method for the manufacture of drug permeable materials.

(30) Priority : **21.01.88 JP 9479/88**

(43) Date of publication of application :
**02.08.89 Bulletin 89/31**

(45) Publication of the grant of the patent :
**08.04.92 Bulletin 92/15**

(84) Designated Contracting States :
**CH DE FR GB IT LI**

(56) References cited :
**EP-A- 0 219 076**
**GB-A- 2 191 397**
**Patent Abstracts of Japan, unexamined appli-
cations, C field, vol. 9, no. 278, November 6,
1985 The Patent Office Japanese Government
page 22 C 312**

(56) References cited :
**Patent Abstracts of Japan, unexamined appli-
cations, C field, vol. 11, no. 13, January 14,
1987 The Patent Office Japanese Government
page 155 C 397**

(73) Proprietor : **Dow Corning Kabushiki Kaisha
15-1, Nishishimbashi 1-chome Minato-ku
Tokyo 105 (JP)**

(72) Inventor : **Urabe, Yosuke
2-10-14, Fujimino
Hiratsuka-shi Kanagawa-gen (JP)**
Inventor : **Kawashima, Hiroyuki
3-10-1, Terao Kita
Ayase-shi Kanagawa-gen (JP)**

(74) Representative : **Lewin, John Harvey et al
Elkington and Fife Prospect House 8
Pembroke Road
Sevenoaks, Kent TN13 1XR (GB)**

## Description

The present invention relates to a method for the manufacture of drug-permeable materials used in a drug delivery system and are suitable for the formation of such systems which can release drugs gradually over time. Such systems may be of the transdermal drug delivery type or of a type which is implanted either subcutaneously or in a body cavity.

At present, typical drug-administering agents that allow gradual release of drugs over time can be divided into two groups, the so called matrix type consisting of a base material mixed with the drug, and the so-called reservoir type in which a separate drug reservoir layer is covered by a drug permeable polymeric material.

In recent years, silicone rubbers have been used as drug permeable materials in such systems because silicone rubbers generally have high affinity for many drugs and excellent drug permeability, while avoiding unwanted effects on the human body.

However, a drug permeable material consisting of a silicone rubber, while exhibiting excellent permeability or releasability with oleophilic drugs, does not usually show good permeability or drug releasability to hydrophilic drugs.

In the effort to promote the permeability or releasability of silicone rubber drug permeable materials with hydrophilic drugs, the addition of hydrous water-soluble additives has been attempted. Examples of such proposals are seen in Japanese Kokai Patents Nos. Sho 59[1984]-44310, Sho 59[1984]-104314, and Sho 60[1985]-123416.

In these techniques, however, the addition of water soluble additives compromises or decreases the desirable characteristics of silicone rubbers. These techniques, therefore, do not necessarily offer satisfactory drug-permeable materials.

In addition, the drug permeable materials to which water soluble additives have been added tend to have increased volumes because the water soluble additives absorb water. When a drug administering agent with such a drug permeable material is implanted in a patient's body, this swelling of the drug administering agent can create pressure against the affected tissues or organs.

JP-A-61-191609 discloses a slow-release medicinal preparation made by Freezing an aqueous solution of a polyvinyl alcohol containing a pharmacologically active substance.

An object of the present invention is to offer a method for easily manufacturing excellent drug permeable materials free from the above stated problems and which can be used as the base materials for matrix type drug administering agents, or for forming a drug permeable layer of reservoir type drug administering agents.

The present invention provides a method for the manufacture of a drug-permeable material from a mixture of a curable silicone rubber and water-containing polyvinyl alcohol, wherein said polyvinyl alcohol has a degree of polymerization of at least 1000 and a degree of saponification of at least 95 mole per cent, the method comprising

(A) curing the silicone rubber component of said mixture and

(B) subjecting the mixture to a low-temperature treatment at a temperature below -5°C to cause gelation of said water-containing polyvinyl alcohol, the water-containing polyvinyl alcohol gel having a water content of from 70 per cent to 95 per cent by weight, and constituting from 5 to 50 per cent by weight of said mixture.

In the present invention, a mixture of a curable silicone rubber and a water-containing polyvinyl alcohol (hereafter "water-containing PVA"), is subjected to a treatment which cures the silicone rubber and causes low temperature (ie, below -5°C) gelation of the water-containing PVA. The resultant permeable materials are used to manufacture drug delivery systems.

The drug permeable material manufactured by the abovementioned method consists of a homogenous mixture of a water-containing gel of PVA, which has been crystallized and gelated by a low temperature treatment, and a cured silicone rubber. Because the water-containing gel of PVA contains a large quantity of water, the material exhibits excellent permeability or releasability of drugs even when the drugs are hydrophilic. In addition, because the PVA has been crystallized with a low temperature treatment, the silicone rubber, not having been exposed to the unfavorable effect of the water in PVA, retains most of its desirable characteristics. Furthermore, the material does not experience a volume increase during use.

A further advantage of the materials of this invention is that control of the degree or rate of the release of a hydrophilic drug can be achieved by adjusting the proportion of the water-containing PVA gel present in the composition.

The above stated excellent effects of the present invention may be explained in the following manner. The hydrous PVA gel dispersed in the silicone rubber constitutes channels that transfer the hydrophilic drug at a high efficiency, thus providing a high drug permeability. In addition, because the hydrous PVA gel has been crystallized at a low temperature, the water contained in the gel does not affect the silicone rubber in an unfavorable manner.

The present invention will be explained more specifically below.

In a typical example of the present invention, an aqueous solution of PVA is prepared by dissolving PVA powder in water at high temperature using an autoclave, for example. The aqueous solution of PVA is added to a curable silicone rubber and mixed. After further addition and mixing of the silicone rubber, curing agent and some other additives as required, a composition is prepared. The additives to be used here may be surfactants, and water soluble additives. Next, the composition is subjected to a silicone curing treatment to cause the silicone rubber component to be crosslinked. In addition, by subjecting the whole composition to a low temperature treatment, the hydrous PVA is gelated in such a way that a crystallized hydrous gel is formed. As the result of these treatments, a drug permeable material is obtained.

The drug permeable material of the present invention is suitable for producing a drug administering material. If the drug permeable material of the present invention is to be used as the base of a matrix type drug administering agent, the drug can be mixed with the mixture of PVA and silicone rubber before they are cured and low temperature gelated. In this case, the drug is contained in the base material in a dispersed state or dissolved state.

In carrying out the above process, any of various curable silicone rubbers may be used. However, considering the low heat resistance of drugs, it is desirable that a room temperature curable silicone rubber should be adopted. The specific examples of such silicone rubbers may be addition polymerization type millable silicone rubbers, addition polymerization type liquid silicone rubbers, dealcoholating condensation reaction type liquid silicone rubbers, and others. However, the types of silicone rubbers are not limited to these examples.

Any PVA with a degree of polymerization of 1000 or higher, and a degree of saponification of 95 mol per cent or higher can be used in the method of this invention with favorable results. The water-containing PVA gel should have a water content within a range of 70% to 95% by weight, or preferably 80% to 90% by weight. If the water content is lower than 70%, a good drug permeability may not be obtained with hydrophilic drugs.

With regard to the drugs, any of them may be used with the drug permeable materials of the present invention without requiring particular limitations. Although the drug permeable materials of the present invention show excellent permeability with hydrophilic drugs, they also show good permeability with oleophilic drugs, and are able to be used with them.

The specific examples of the hydrophilic drugs with which the drug-permeable materials of the present invention can be used with favorable results are as follows:

Cough suppressants:

Codeine phosphate, dihydrocodeine phosphate, ephedrine chloride, dextromethorphan hydrobromide.

Narcotics and analgesics:

Morphine hydrochloride, oxycodone hydrochloride, hydromorphone hydrochloride, naloxone hydrochloride, meperidine hydrochloride.

Analgesics and antipyretics:

Aspirin, phenacetin, sodium salicylate, acetaminophen, antipyrine, aminopyrine.

Analgesics and anti-inflammatories:

Indomethacin, oxyphenbutazone, salicylic acid, ibuprofen, mefenamic acid, ketoprofen, flufenamic acid, acetaminophen, phenylbutazone.

Antihistaminics:

Diphenhydramine hydrochloride, pheniramine maleate, chlorpheniramine maleate.

Sedatives and tranquilizers:

Bromodiethylacetylurea, chlorpromazine hydrochloride, thioridazine hydrochloride, acepromazine maleate, meprobamate, imipramine hydrochloride, diazepam.

Antidepressants:

Imipramine hydrochloride, amitriptyline hydrochloride.

Hypnotics:

Phenobarbital sodium, amobarbital sodium, barbital sodium.

Anticholinergics (drugs to control motion sickness):

Scopolamine hydrochloride, promethazine hydrochloride.

Anticonvulsants and antiepileptics:

Ethotoin, trimethadione, ethosuximide.

Antibiotics:

Penicillin, fradiomycin sulfate [neomycin bisulfate], erythromycin, chloramphenicol, tetracycline, gentamicin.

Antiseptics and antimicrobials:

Benzalkonium chloride, nystatin, nitrofurazone, N′-acetylsulfanilamide.

Anesthetics:

Lidocaine hydrochloride, dibucaine hydrochloride, procaine hydrochloride.

Antiarrhythmics:

Quinidine sulfate, procaine hydrochloride, lidocaine hydrochloride.

Antihypertensives:

Clonidine hydrochloride, propranolol hydrochloride, oxyprenolol hydrochloride, alprenolol hydrochloride, bupranolol hydrochloride.

Antiglaucoma agents:

Pilocarpine hydrochloride.

Vitamins:

Riboflavin phosphate, ascorbic acid, thiamine hydrochloride, pyridoxine hydrochloride.

Diuretics:

Acetazolamide, hydrochlorothiazide.

In the process preparing the drug permeable material of the present invention, the silicone rubber and hydrous PVA can be mixed by any mixing means desired. The curing of silicone rubber should be carried out under the conditions as required by the type of silicone rubber employed, such as in a saturated steam below 100°C, in a room temperature atmosphere, or under other conditions depending on the particular curing system employed. It is generally favored that the silicone rubber should contain a curing catalyst. Examples of such catalysts are platinic chloride and other platinum compounds, tin compounds such as stannous octoate, stannous oleate, or dimethyltindireodecanoate, carboxylic acid metal salts, and others.

The low-temperature gelation of water-containing PVA is usually carried out by a freezing treatment of the composition at temperatures below -5°C for 5 or more hours and by the leaving of the composition at temperatures below 10°C for the next 10 or more hours. This low temperature treatment results in the crystallization and gelation of the PVA.

In the composition consisting of hydrous PVA and silicone rubber, the amount of hydrous PVA should constitute 5 to 50 weight per cent, or preferably 10 to 30 weight per cent of the sum of the hydrous PVA and silicone rubber. If the amount of the hydrous PVA is less than 5 weight per cent of the sum of the hydrous PVA and silicone rubber, the resultant drug permeable material will have a low permeability or releasability of hydrophilic drugs, thus making it difficult for the material to meet the proposed effect requirements satisfactorily. The upper limit of the amount of the hydrous PVA cannot be set specifically because it should vary depending on the type of the drug, the desired drug releasing rate, and other factors. In general, however, if the percentage of the hydrous PVA in the sum of the PVA and silicone rubber exceeds 50 weight per cent, the initial release of the drug will become conspicuously high. In addition, because of the relative increase in the moisture content, the drug-administering agent will release much moisture during storage. The resulting excessively high viscosity of the agent can then affect the workability of the agent at the time of the component mixing.

In the preparation of the hydrous PVA/silicone rubber composition, there is no particular restriction as to which of the two treatments, the silicone rubber curing treatment or the hydrous PVA low temperature treatment, should precede the other. It is allowable that the curing of silicone rubber should be carried out after the low temperature treatment of PVA. However, in that particular case, sometimes silicone rubber curing may not proceed smoothly. For this reason, it is preferable that the curing of the silicone rubber precedes the low temperature treatment of hydrous PVA.

EXAMPLES

The application examples of the present invention will be explained below.

Example 1

A PVA powder, degree of polymerization 1750, degree of saponification 99.5 mol% or higher, was dissolved in an autoclave at a temperature of 120°C for 20 minutes to obtain an aqueous solution of PVA with a water content of 85%. Then, 20 g of the obtained aqueous solution of PVA were mixed with 80 g of the "Silastic (R) 382 medical grade elastomer", a room-temperature curing-type silicone rubber elastomer. Next, 200 mg of yellow dye as a substitute drug and 0.5 g of the curing catalyst for the elastomer were added and mixed with the mixture to obtain a composition.

This composition was then press molded into a sheet of 1 mm thickness. It was cured by being left at room temperature for a day. Later, it was frozen at -20°C for 10 hours, and then left at 2°C for 10 hours so that a sample could be obtained by the crystallization and gelation of the PVA.

Example 2

A sample was prepared by repeating the process of Example 1, except that 20 g of the aqueous solution of PVA with a moisture content of 85% were replaced by 20 g of PVA with a moisture content of 90%.

Comparative Example 1

A sample was prepared by repeating the process of Example 1 except that 20 g of the aqueous solution of PVA were replaced by 20 g of lactose to obtain a sample. In this example, however, low temperature treatment was not carried out (as it was not in all the following comparative examples).

Comparative Example 2

A sample was prepared by repeating the process of Example 1, except that 20 g of the aqueous solution of PVA were replaced by 20 g of glycerin.

Comparative Example 3

A sample was prepared by repeating the process of Example 1, except that the mixing of 20 g of the aqueous solution of PVA with silicone rubber was replaced by direct mixing of 20 g of the powder of the same PVA as used in Example I with silicone rubber.

### Comparative Example 4

A sample was prepared by repeating the process of Example 1 except for the elimination of the use of the aqueous solution of PVA, the increase of the amount of the silicone rubber to 100 g, and the change of the amount of curing catalyst to 0.6 g.

The samples obtained in the above Examples 1 and 2 and Comparative Examples 1-4 were subjected to the measurements of the drug release percentage, tensile strength, elongation and the volume increase percentage using the procedures described below.

### Drug releasing percentage

A sample, cut to a size of 140 mm x 13 mm (width), is rolled and placed in an Erlenmeyer flask. Then, after 35 g of water are added, the flask is stopped and its content is stirred with a magnetic stirrer for 24 hours to release the drug. Later, the sample is removed out of the water. If the solution left is too turbid, it is filtered. The obtained aqueous solution is then used as the drug-released sample.

Next, the drug released sample is compared with a calibration curve prepared with the filters of a photoelectric colorimeter. If the linear region of the calibration curve is applicable to the drug released sample, the sample is directly subjected to the measurement of the transmittance of light. If not, the sample is diluted until applicable. The sample thus obtained is subjected to the measurement of the transmittance of light. From the transmittance the absorbance is calculated. From the absorbance, the solution concentration of the drug released sample and the amount of the released drug are calculated.

$$\text{Light transmittance (t)} = I/I_o$$
$$\text{Light absorbance (A)} = \log I_o/I$$

(where $I_o$ is the intensity of the incident light and $I$ is the intensity of the transmitted light.)

### Tensile strength and elongation

Tensile strength and elongation of the sample material are measured according to section 3. of JIS K6301 (Physical Testing Methods on Vulcanized Rubbers). The shape of the test piece from the sample should be the dumbbell shape No. 2 specified in JIS K6301, 3.2.1.

### Volume increase percent

The sample after the drug release is subjected to the measurement of the volume. From the volume values before and after the drug release, the volume increase percentage is calculated.

The results of these measurements are shown in Table I.

### Example 3

A sample was prepared by the same process as that of Example 1 except for the changes of the amount of silicone rubber from 80 g to 70 g, the amount of aqueous solution of PVA from 20 g to 30 g, and the amount of curing catalyst from 0.5g to 0.45 g.

### Example 4

A sample was prepared by the same process as that of Example 1 except for the changes of the amount of silicone rubber from 80 g to 50 g, the amount of the aqueous solution of PVA from 20 g to 50 g and the amount of curing catalyst from 0.5 g to 0.3 g.

The samples obtained in Examples 3 and 4 were subjected to the calculation of the drug release percentage by the method described previously. The results are shown in Table II together with those of Example 1 and Comparative Example 4

### Examples 5-6 and Comparative Examples 5-6

Samples were prepared by the same process as that of Example 1 except that the formulas shown in Table III were used. The silicone rubber elastomer used in Example 1 was used in these examples. The aqueous solution of PVA with a moisture content of 85% was used. Pyridoxine hydrochloride was used as the drug in these examples.

6

In these examples, pyridoxine hydrochloride was determined by the method for determining pyridoxine hydrochloride injection solution (Japanese Pharmacopoeia, Vitamin B6 injection solution). This method proceeds as follows. From the drug-released sample obtained by the previously described drug-release percentage measurement method, a volume corresponding to about 0.02 g of pyridoxine hydrochloride is taken by measurement. Then water is added to the sample to make up 100 ml. Next, 25 mL of this solution is taken, and, water is added another time to make up to 200 ml of a sample solution. Separately, the standard pyridoxine hydrochloride is dried in a desiccator for 4 hours. Then about 0.1 g of the dried pyridoxine hydrochloride is precisely measured, and water is added to make up to 100 ml. Next, taking 5 ml of this solution by measuring, water is added another time to make up to 200 ml. The resultant solution is regarded as the standard solution. Now, 2.0 ml of barbital buffer, 9.0 ml of isopropanol and 2.0 ml of an ethanol solution of 2,6-dibromoquinonechlorimide are added to 1 ml of the sample solution, and the mixture is thoroughly shaken. Then isopropanol is added another time to make up to 25 ml. After this solution is left standing for 90 minutes, it is subjected to the measurement of absorbance Ar at a wavelength of 650 nm using as control the solution obtained by the same process as described above excepting that 1 ml of water is used in place of the sample solution. Separately, 1 ml of the standard solution is taken by measurement, and after it had also undergone the same process as described above, the resultant solution is subjected to the measurement of absorbance As. The amount of pyridoxine hydrochloride, M (mg) is calculated from the equation shown below.

$$M \text{ (mg)} = \text{Amount (mg) of standard pyridoxine hydrochloride} \times Ar/As \times 1/5$$

With the samples obtained in Examples 5 and 6 and Comparative Example 5 and 6, the drug release percentages were calculated by the method described above. The results are shown in Table IV.

Examples 7-9 and Comparative Example 7

Samples were prepared by the same process as that of Example 1 except that the formulas shown in Table V were used. In these examples, the silicone rubber used was the same silicone rubber elastomer used in Example 1; the aqueous solution used had a moisture content of 0.85%; and the drug used was pilocarpine hydrochloride. With the samples obtained in Examples 7-9 and Comparative Example 7, the drug-release percentages were calculated by using the previously described method. The results are shown in Table VI.

Example 10 and Comparative Example 8

Samples were prepared by the process of Example 1 except for the use of the formula shown in Table VII. In these examples, the silicone rubber was the same as the one used in Example 1; the aqueous solution of PVA was 85%, and the drug used was quinidine sulfate.

The samples obtained by Example 10 and Comparative Example 8 were subjected to the measurement of drug release percentage. The results are shown in Table VIII.

In addition, with the samples obtained in Examples 1 and 3 and Comparative Example 4, cumulative release (in mg) was observed with time. The results are shown in Table IX.

Table I.

| | Drug release (%) | Tensile strength $(kg/cm^2)$ | Elongation (%) | Volume increase (%) |
|---|---|---|---|---|
| Example 1 | 5.2 | 17.5 | 180 | 0 |
| Example 2 | 8.6 | 18.0 | 190 | 0 |
| Comparative Example 1 | 4.1 | 9.3 | 150 | +35 |
| Comparative Example 2 | 9.4 | 4.9 | 430 | +135 |
| Comparative Example 3 | 3.5 | 10.2 | 140 | +10 |
| Comparative Example 4 | 1.7 | 24.3 | 180 | 0 |

Table II.

| | Amount of hydrous PVA | Drug release percentage |
|---|---|---|
| Example 1 | 20 wt % | 5.2 |
| Example 3 | 30 wt % | 15.9 |
| Example 4 | 50 wt % | 44.7 |
| Comparative Example 4 | -- | 1.7 |

Table III

|  | Example | | Comparative Example | |
|---|---|---|---|---|
|  | 5 | 6 | 5 | 6 |
| Silicone Rubber | 80g | 70g | 100g | 80g |
| Aqueous Solution of PVA | 20g | 30g | -- | -- |
| Drug | 200mg | 200mg | 200mg | 200mg |
| Catalyst | 0.5g | 0.45g | 0.6g | 0.5g |
| Lactose | -- | -- | -- | 20g |

Table IV

|  | Pyridoxine Hydrochloride Release Percentage |
|---|---|
| Example 5 | 11.6 |
| Example 6 | 20.2 |
| Comparative Example 5 | 4.5 |
| Comparative Example 6 | 6.6 |

Table V.

|  | Example | | | Comparative Example |
|---|---|---|---|---|
|  | 7 | 8 | 9 | 7 |
| Silicone Rubber | 80g | 70g | 50g | 100g |
| Aqueous Solution of PVA | 20g | 30g | 50g | -- |
| Drug | 2g | 2g | 2g | 2g |
| Catalyst | 0.5g | 0.45g | 0.3g | 0.6g |

Table VI.

|  | Pilocarpine Hydroxide Release Percentage |
|---|---|
| Example 7 | 19.0 |
| Example 8 | 51.7 |
| Example 9 | 91.8 |
| Comparative Example 7 | 7.6 |

Table VII.

|  | Example 10 | Comparative Example 8 |
|---|---|---|
| Silicone Rubber | 70g | 100g |
| Aqueous Solution of PVA | 30g | -- |
| Drug | 10g | 10g |
| Catalyst | 0.45g | 0.6g |

Table VIII.

|  | Quinidine Hydrochloride (%) |
|---|---|
| Example 10 | 35.0 |
| Comparative Example 8 | 2.8 |

11

**Table IX.**

| Time (hr) | Example 1 | Example 3 | Comparative Example 4 |
|---|---|---|---|
| 0.5 | 0.090 | 0.075 | 0.045 |
| 1 | 0.096 | 0.088 | 0.048 |
| 2 | 0.109 | 0.105 | 0.052 |
| 4 | 0.124 | 0.131 | 0.056 |
| 6 | 0.136 | 0.157 | 0.062 |
| 12 | 0.199 | 0.222 | 0.061 |
| 24 | 0.220 | 0.316 | 0.072 |

## Claims

1. A method for the manufacture of a drug-permeable material from a mixture of a curable silicone rubber and water-containing polyvinyl alcohol, wherein said polyvinyl alcohol has a degree of polymerization of at least 1000 and a degree of saponification of at least 95 mole per cent, the method comprising
(A) curing the silicone rubber component of said mixture and
(B) subjecting the mixture to a low-temperature treatment at a temperature below -5°C to cause gelation of said water-containing polyvinyl alcohol, the water-containing polyvinyl alcohol gel having a water content of from 70 per cent to 95 per cent by weight, and constituting from 5 to 50 per cent by weight of said mixture.

2. A method according to Claim 1, wherein the water-containing polyvinyl alcohol gel has a water content of from 80 per cent to 90 per cent by weight.

3. A method according to Claim 1, wherein the water-containing polyvinyl alcohol gel constitutes from 10 to 30 per cent by weight of the mixture.

4. A method according to Claim 1, wherein said silicone rubber component is cured by a curing agent selected from platinum compounds, tin compounds and carboxylic acid metal salts.

5. A method according to Claim 1, wherein a hydrophilic drug is incorporated in the mixture to form a matrix type drug administering agent.

6. A method according to Claim 1, wherein the resulting drug-permeable material is used to cover a separate drug reservoir layer to form a reservoir type drug administering agent.

## Patentansprüche

1. Verfahren zum Herstellen eines arzneimitteldurchlässigen Materials aus einer Mischung eines härtbaren Siliconkautschuks und wasserhaltigem Polyvinylalkohol, wobei der Polyvinylalkohol einen Polymerisationsgrad von mindestens 1000 und einen Verseifungsgrad von mindestens 95 Mol % aufweist, durch

(A) Härten des Siliconkautschukbestandteils der Mischung und

(B) Aussetzen der Mischung einer Tieftemperaturbehandlung bei einer Temperatur unter -5°C, um Gelieren des wasserhaltigen Polyvinylalkohols auszulösen, wobei das wasserhaltige Polyvinylalkoholgel einen Wassergehalt von 70 bis 95 Gew % aufweist und von 5 bis 50 Gew % der Mischung ausmacht.

2. Verfahren nach Anspruch 1, **dadurch gekennzeichnet,** daß das wasserhaltige Polyvinylalkoholgel einen Wassergehalt von 80 Gew % bis 90 Gew % aufweist.

3. Verfahren nach Anspruch 1, **dadurch gekennzeichnet**, daß das wasserhaltige Polyvinylalkoholgel von 10 bis 30 Gew % der Mischung ausmacht.

4. Verfahren nach Anspruch 1, **dadurch gekennzeichnet**, daß der Siliconkautschukbestandteil durch ein Härtungsmittel gehärtet wird, ausgewählt aus Platinverbindungen, Zinnverbindungen und karbonsauren Metallsalzen.

5. Verfahren nach Anspruch 1, **dadurch gekennzeichnet**, daß ein hydrophiles Arzneimittel in die Mischung eingeschlossen ist, um eine Verabreichungsform des Matrixtyps des Arzneimittels auszubilden.

6. Verfahren nach Anspruch 1, **dadurch gekennzeichnet**, daß das erhaltene, arzneimitteldurchlässige Material verwendet wird, um eine getrennte Arzneimittelvorratsschicht abzudecken, um eine Verabreichungsform des Vorratstyps des Arzneimittels auszubilden.


**Revendications**

1. Un procédé pour la fabrication d'une matière perméable à un médicament à partir d'un mélange de caoutchouc de silicone durcissable et d'alcool polyvinylique contenant de l'eau, où ledit alcool polyvinylique a un degré de polymérisation d'au moins 1000 et un degré de saponification d'au moins 95 moles pour cent, le procédé consistant à

(A) faire durcir le composant caoutchouc de silicone dudit mélange, et

(B) soumettre le mélange à un traitement à basse température à une température inférieure à -5°C pour provoquer la gélification dudit alcool polyvinylique contenant de l'eau, le gel d'alcool polyvinylique contenant de l'eau ayant une teneur en eau de 70 pour cent à 95 pour cent en poids, et constituant 5 à 50 pour cent en poids dudit mélange.

2. Un procédé selon la revendication 1, dans lequel le gel d'alcool polyvinylique contenant de l'eau a une teneur en eau de 80 pour cent à 90 pour cent en poids.

3. Un procédé selon la revendication 1, dans lequel le gel d'alcool polyvinylique contenant de l'eau constitue 10 à 30 pour cent en poids du mélange.

4. Un procédé selon la revendication 1, dans lequel ledit composant caoutchouc de silicone est durci par un agent durcisseur choisi parmi les composés du platine, les composés de l'étain et les sels métalliques d'acides carboxyliques.

5. Un procédé selon la revendication 1, dans lequel un médicament hydrophile est incorporé au mélange pour former un agent d'administration de médicament du type à matrice.

6. Un procédé selon la revendication 1, dans lequel la matière perméable à un médicament résultante est utilisée pour recouvrir une couche de réservoir de médicament séparée pour former un agent d'administration de médicament du type à réservoir.